# EUROPEAN PATENT APPLICATION

(11) **EP 4 388 987 A1**
(43) Date of publication of application: **26.06.2024**
(21) Application number: 23217200.7
(22) Date of filing: 15.12.2023
(51) Int. Cl.: A61B 5/00

(54) **A SENSING DEVICE AND A METHOD FOR DETECTION OF A CHARACTERISTIC OF A SUBSTANCE AT MULTIPLE TIME POINTS**

(30) Priority: 22.12.2022 EP 22216128
(71) Applicant: Imec VZW, 3001 Leuven (BE)
(72) Inventor: LAGAE, Liesbet, 3000 Leuven (BE); HENRY, Olivier, 1950 Kraainem (BE); VAN HELLEPUTTE, Nick, 3060 Korbeek-Dijle (BE)
(74) Representative: AWA Sweden AB

(57) **Abstract**

A sensing device comprising:
a plurality of cavities (110), each comprising an opening;
a plurality of sensors (120) for detecting the at least one characteristic, the plurality of sensors being arranged into a plurality of sets of sensors, each set being arranged in a mutually unique cavity;
a plurality of protective membranes (130), each being arranged to cover the opening of the mutually unique cavity (110), preventing the substance from entering the cavity, thereby protecting the set of sensors from being exposed to the substance.

The sensing device is configured for providing a different activation timing for different protective membranes (130), whereby different sets of sensors (120) are exposed to the substance at different points in time, for providing detection of the at least one characteristic at multiple time points.

## Description

### Technical field

The present description relates to a device and a method for monitoring a process, and more specifically to a sensing device, a capsule, a system, and a method for detection of at least one characteristic of a substance at multiple time points.

### Background

Monitoring of substances undergoing chemical and/or physical processes is applicable in a vast variety of applications within industrial fields as well as within research. For example, pharmaceutical process analysis is frequently used for monitoring production batches in production of medicines. Other application areas where frequent substance monitoring is of interest include environmental studies such as monitoring of levels of pollutants in the air, and monitoring levels of toxins and pH in water and soil, as well as monitoring levels of contamination in food.

For example, within the field of life sciences, cell therapy, which uses human cells to restore, maintain, or improve the functioning of human tissues or organs, has become one of the fastest-growing segments. Cell-based therapy is rapidly developing and is greatly expanding the opportunities to treat a vast array of clinical disorders, mostly focused but not limited to cancer. However, one of the barriers is the manufacturing, since cells are notoriously unpredictable to grow.

Conventional pharmaceutical process analytics are designed to monitor production batches which are all chemically identical, and hence are followed up via a limited range of chemical and physical characteristics such as pH, temperature and dissolved gases. On the other hand, new medicines such as biological medicine and immune therapies, as for example those using precisely engineered cells, that aim to modulate the immune system need to be biologically tested a few times per day to ensure that these cultures are kept under ideal growth conditions and that cells retain their potency. Centralized manual sampling and testing are the current practices when only a few bioreactors need to be followed.

The emerging field of cell and gene therapy, which uses human cells to restore, maintain, or improve the functioning of the immune system, of human tissues or organs, has become one of the fastest-growing segments. Cell and gene based therapy is rapidly developing and is greatly expanding the opportunities to treat a vast array of clinical disorders, mostly focused but not limited to cancer. Cell and gene therapies can use cell cultures, or primary cells from another patient (e.g. allogenic) or the patient itself (autologous). However, one of the barriers is to ensure tight process control and safe manufacturing, since cells are notoriously unpredictable to grow. When done in centralized facilities, conventional lab-centralized lab sampling is still possible. However, this does not scale with the need of millions of cancer patients that need their personalized dose to be generated and monitored correctly.

Chemical parameters such as pH, temperature, and dissolved oxygen (DO) are typically followed online using existing measurement probes through bioreactor ports. Biological parameters such as those related to metabolic activity i.e. glucose or lactate are typically measured at regular intervals e.g. hourly, and protein samples should be taken at least daily. Frequent measurements are important for monitoring the quality and growth of biological medicines as well as for monitoring of potential contaminants in the batch.

Conventional biosensors do not match the need of in situ biomonitoring. One problem with current biosensors is that one-time-use reagents are needed for biosensing reactions to occur and to create electronic signals. Thus, a conventional biosensor is not suited for multiple measurements over time. Other problems with conventional biosensors are degradation over time when exposed to the process under investigation, and sensor drift over time requiring frequent recalibration of the sensor.

Hence, there is a need in the art for further improvements related to sensors for monitoring of substances undergoing chemical and/or physical processes.

### Summary

An objective of the present description is to mitigate, alleviate or eliminate one or more of the above-identified deficiencies in the art and disadvantages singly or in any combination. These and other objectives are at least partly met by the invention as defined in the independent claims. Preferred embodiments are set out in the dependent claims.

According to a first aspect there is provided a sensing device for detection of at least one characteristic of a substance, the sensing device comprising:
a plurality of cavities, wherein each cavity of the plurality of cavities comprises an opening;
a plurality of sensors configured for detecting the at least one characteristic of the substance, wherein the plurality of sensors is arranged into a plurality of sets of one or more sensors, and wherein each set of one or more sensors is arranged in a mutually unique cavity of the plurality of cavities;
a plurality of protective membranes, wherein each protective membrane of the plurality of protective membranes is arranged to cover the opening of the mutually unique cavity, so as to prevent the substance from entering the cavity, thereby protecting a mutually unique set of one or more sensors of the plurality of sets from being exposed to the substance;
wherein the sensing device is configured for providing a different activation timing for different protective membranes among the plurality of protective membranes, to allow the set of one or more sensors protected by the different protective membranes to be exposed to the substance, whereby different sets of one or more sensors are exposed to the substance at different points in time, for providing detection of the at least one characteristic of the substance at multiple time points.

By the term "substance" is here meant any physical material or matter with a particular physical and/or chemical constitution with particular characteristics. The substance may be in the form of a solid, a liquid, or a gas, or a combination thereof. However, in the present context, the substance typically has some fluid properties, such as a liquid or a gas, or a liquid comprising portions of solid material.

The substance may be a biological material. By way of example, the biological material may be in the form of a liquid comprising also portions of solid substance. For example, a biological material may be a bodily fluid of a human or an animal, wherein the solid substance may be in the form of cells in the liquid. By way of further example, the biological material may be a production batch of a production of medicines such as biological medicine and immune therapies with precisely engineered cells that aim to modulate the immune system. In both medical research and pharmaceutical production there may be needs of monitoring certain chemical or physical characteristic over time, wherein the sensing device may be applied.

Characteristics of interest to monitor in processes of biological material may be characteristics related to metabolic activity, such as glucose or lactate concentrations, or it could be characteristics such as concentrations of certain proteins in the substance. Monitoring of such parameters may provide information on product quality of the biological medicine, information about potential contaminations, and information on growth. Other characteristics of interest may be a pH, a temperature, a dissolved gas, a cell, a biomarker, a metabolite, or a molecular species.

However, the substance is not limited to being a biological material. The substance may alternatively be non-biological material such as ambient air, or lake or sea water, or soil. For example, a sensing device may be arranged on an exterior of a vehicle, to periodically measure the level of pollutants in the ambient air. By way of further example, the sensing device may be applied in environmental studies such as monitoring levels of toxins or pH in water or soil. Yet another alternative is to apply the sensing device for monitoring contamination of food.

By the term "membrane" is here meant any element forming a thin layer, plane or film and functioning as a boundary or a barrier separating two environments by preventing passage of a substance between the two environments. In the present disclosure, the membrane is typically a thin film arranged to cover the opening of the cavity, thereby separating the environment exterior to the cavity from the environment interior to the cavity. By way of example, the membrane may be a freestanding thin film. By way of further example, the membrane may comprise a metal. The membrane may comprise a metal layer or a patterned metal structure as part of a non-metallic thin film. Given as a non-limiting example, the metal may be activated to controllably disrupt the membrane and enable the substance to enter the cavity, thereby exposing the sensor to the substance.

The opening of the cavity, and thus also the cavity, may be provided with any suitable cross-sectional shape. By way of example, the cavity may have a circular, elliptical, square, or rectangular shape. However, it is preferred that the cavity has an oblong cross-sectional shape. Preferably, the cavity may have a cross-sectional width of about 100 µm and a cross-sectional length of about 200 µm.

Given only as an example, the cavity may have a depth in the range of 200 µm to 500 µm, and preferably a depth of about 400 µm.

Further, the cavity surface may be made hydrophilic to enable wetting. By way of example, in order to increase hydrophilicity, the cavity may be coated with polyethylene glycol (PEG), or proteins such as bovine serum albumin, or any other molecule known to induce a low contact angle. By the present arrangement, homogeneous filling of the cavity may be facilitated.

A sensor may be, but is not limited to, a biosensor. A biosensor is an analytical sensor for detection of a chemical compound, an analyte. The biosensor combines a biological component, a reagent, with a physicochemical sensor. The reagent may be, but is not limited to, enzymes, antibodies, DNA, RNA, aptamers, supramolecular scaffolds, or molecularly imprinted polymers. The reagent may be a biologically derived material capable of binding with the analyte under study. Resulting from the interaction of the analyte with the reagent, the sensor converts one signal into another signal, typically an electrical signal, to quantify the analyte. Reagents may be pre-immobilized on the sensor surface or may be provided to the sensor when needed.

Given as a non-limiting example, the sensor may have a size, such as a diameter, in the range of 100-200 µm.

Each sensor is typically a sensor capable of providing only a single measurement, a one-time-use sensor. Especially biosensors comprising reagents may only be used for a single measurement or for a limited period of time until the reagent is consumed upon exposure to the substance or until the surface of the sensor is no longer responding, i.e. due to saturation of the sensor.

However, by providing a plurality of sets of one or more sensors, wherein each set is protected by a mutually unique protective membrane, and by providing different activation timing for different protective membranes, the sensing device may be reusable for multiple measurements by using for example a single set of sensors for each measurement event. By the present arrangement, a sensing device that may be able to monitor a characteristic by making frequent measurement over a period of time, such as over days or months, may be provided.

Each set comprises at least one sensor. It should be realized that a set may comprise more than one sensor. Different sensors in the set may thus be used for detecting different characteristics of the substance. However, it should be realized that plural (such as all) sensors in the set may be used for detecting a same characteristic of the substance to provide redundancy of sensors.

By way of example, the sensing device may be used in a bioreactor or cell culture, for monitoring of a biological process such as in production of vaccines, antibody medicine and cell therapies. Activation may sequentially trigger the sensors such that the plurality of one-time-use sensors are used only once when required. Upon activation the substance to be analyzed and potentially the reagents are brought together to an active area of the sensor. The sensing device comprises a plurality of sets of sensors for performing plural sensing events at a plurality of points in time. The present arrangement allows for creating in situ autonomous measurements at different points in time with read out of at least one characteristic of the substance.

It should be understood that the sensing device may be configured to detect more than one characteristic of the substance. Given as a non-limiting examples, the sensing device may be configured for detection of the level of 8 different proteins in the substance at 100 different points in time. Such a sensing device would require at least 800 sensors that are individually activated.

The sensing device may be integrated on an Si substrate. Si substrates may allow for providing a compact sensing device. Si substrates may contain integrated circuits that allow for the actuation of the protective membranes as well as the read out of the different sensors.

By way of example, the protective membranes as well as the sensors may be directly integrated into integrated circuits (IC). Such an arrangement may be particularly relevant in case the plurality of cavities comprises a large number of cavities, such as thousands of cavities. For example, a CMOS may be postprocessed to include cavities, protective membranes and sensors. By way of example, such a CMOS circuit may be a sensor array IC arranged under a cavity and membrane array IC, arranged on top of the sensor array IC. Further, the arrangement may comprise integrated switch matrices configured to connect to the sensors and protective membranes. The present arrangement provides an elegant and compact manner of handling large numbers of cavities and sensors.

An advantage with the sensing device of the first aspect is that the protective membranes keeping the sensors, and when applicable the reagents, protected until the time of activation. Thus, although the sensor device as such may be exposed to the substance, the individual sensors not yet used are still protected from exposure. Preventing exposure of the sensors to the substance may in turn prevent degradation of the sensor and or the reagent. Thus, by the present arrangement a sensing device with a plurality of sensors wherein the sensing properties of the sensors may remain stable until the time of use of the sensor may be provided. Stable sensors may further provide better and more reliable measurement results.

Another advantage is that, since the long-term stability of the sensors may be ensured, measurements at multiple points in time may be provided to allow frequent monitoring over long time periods.

Another advantage is that the sensing device may be integrated in a compact format that may fit into a small capsule that may be implanted into a body or swallowed. This in turn may provide for easy application of in situ monitoring in the body.

A sensing device with cavities has a specific advantage in the manufacturing of the sensing device. Applying the protective membrane may involve harsh processing steps. Such a harsh environment may risk damaging the sensors, or if a reagent is used, it may risk damaging the reagent. However, the portion with the plurality of cavities and protective membranes may be made separate to the portion with the plurality of sensors, thereby avoiding exposing the sensors or the reagents to the harsh environment. The portion with the plurality of cavities and protective membranes and the portion with the plurality of sensors may subsequently be assembled once the harsh processing steps are finalized. Ultimately, a sensing device with higher quality and better sensing properties may be provided.

Yet another advantage is that some of the sensors may be used to provide calibration information. By way of example, some sensors may be provided without an otherwise needed reagent or with a reagent dosed differently. By the present arrangement calibration curves can be derived from differential time response curve between the different sensors.

According to an embodiment, the sensing device further comprises a plurality of semi-permeable membranes, wherein each semi-permeable membrane of the plurality of semi-permeable membranes is arranged to cover the opening of the mutually unique cavity, wherein each semi-permeable membrane is configured to allow passage of a liquid through the semi-permeable membrane and to prevent passage of a solid substance through the semi-permeable membrane, thereby protecting the mutually unique set of one or more sensors of the plurality of sets from being exposed to the solid substance.

The semi-permeable membrane may be arranged on the inside of the cavity with respect to the protective membrane, or the semi-permeable membrane may alternatively be arranged on the outside of the cavity with respect to the protective membrane.

The semi-permeable membrane may enable filtering based on a smaller size of solid substances than the filtering provided by the opening of the cavity. Thus, the semi-permeable membrane may define pore sizes in the range of 5 µm to 10 µm. For example, in case the substance is a biological material, the solid substance may be in the form of cells in the liquid. Pores of the semi-permeable membrane may selectively prevent the solid substances such as cells from passing the semi-permeable membrane, such that the cells are not allowed to enter the cavity to come in physical contact with the at least one sensor.

By way of example, the size of the cells may range from 1 µm, such as for bacteria, to 100 µm such as for mammalian/plant cells, depending on the application. The size of the pores may thus be tuned depending on the application.

The sensing device enables antifouling protection and solids filtering separate from the at least one sensor. Thus, the protection is not provided at a surface of the at least one sensor but rather prevents undesired solid material to enter through the opening of the cavity. This implies that the antifouling protection and solids filtering may be separately optimized independently of the at least one sensor, such as materials used for the at least one sensor.

The semi-permeable membrane may be formed from a material suitable for forming small pore sizes. The semi-permeable membrane may for instance be formed in silicon.

An advantage with this embodiment is that interference or errors introduced in the measurements by the sensor coming into physical contact with solid substances may be avoided. By providing the semi-permeable membrane to cover the opening of the cavity, protection of the sensor from being exposed to the solid substance may be provided, while still allowing the sensor to be exposed to the liquid. In the manner described above, a sensing device configured to detect a characteristic of the substance by being exposed to the liquid of the substance yet reducing or eliminating interference or errors otherwise introduced by solid substances may be provided.

According to an embodiment, each set of one or more sensors comprises a first sensor configured for detecting the at least one characteristic of the substance, and a second sensor configured for detecting a background signal.

By the present arrangement, the background signal detected by the second sensor may be subtracted from the signal detected of the at least one characteristic of the substance by the first sensor, in order to improve the accuracy of the detection of the characteristic of the substance.

Using the second sensor for detecting the background signal may sometimes be referred to as a negative control. The second sensor may alternatively be configured for a positive control, i.e. detecting a signal from a compound of known concentration present in the substance, that can be used to normalize the readings between sensors of different cavities as well as at different points in time.

An advantage with this embodiment is that a sensing device with a plurality of sensors with improved the accuracy of the detection of the characteristic of the substance may be provided.

According to an embodiment, the sensing device is further configured for providing a same activation timing for at least two protective membranes among the plurality of protective membranes, to allow the set of one or more sensors protected by the at least two protective membranes to be exposed to the substance at a same point in time, for providing detection of the at least one characteristic of the substance simultaneously.

An advantage with this embodiment is that, by using two sets of one or more sensors for detection of the characteristic of the substance at the same time, the measurement may be more reliable. For example, in case one set of one or more sensors should malfunction, a correct detection may still be provided by the other set of one or more sensors.

According to an embodiment, the sensing device further comprises a wireless communication unit configured to:
wirelessly transmit, from the sensing device to an external device, a data set comprising information from at least one detection of the at least one characteristic of the substance.

By way of example, the external device may be a computer, a tablet, or a mobile phone.

An advantage is that the data sets may be wirelessly transmitted from the sensing device and provided to the external unit, enabling remote monitoring and follow-up of the development status of the process in the substance.

Another advantage is that the data sets may be extracted from the substance, without the need for extracting neither a sample of the substance nor the sensing device from the location of the substance.

According to an embodiment, the sensing device further comprises a control unit configured to control the activation timing for each protective membrane, thereby controlling the points in time at which the set of one or more sensors protected by the different protective membranes is allowed to be exposed to the substance.

By way of example, the control unit may be configured to provide an electric signal to the respective protective membranes at the activation timing. The electric signal may be a trigger signal for the protective membrane to open. Alternatively, the electric signal may be an electric voltage applied across the respective protective membranes at the respective activation timings, such that the protective membrane is caused to break.

The control unit may be configured to provide individual and independent electric signals to each respective protective membrane. By the present arrangement, a well-controlled activation timing may be provided for the plurality of protective membranes, with a precise sequential activation.

Further, a control device may be configured for actively adjusting control of the activation timing, dependent on one or more previous measurements from other sensors on the sensing device. Adjustments may comprise adjustment of measurement frequency and/or which protective membranes to activate. Alternatively, if the sensing device is a sensing device comprising different types of sensors configured for detection of different characteristics, the sensing device may switch from detecting a first characteristic to detecting a second characteristic at the next sensing event, based on the value of the previous measurement.

According to an embodiment, the control unit is further configured for allowing programmable control of the activation timing for each protective membrane.

An advantage is that the sequence of measurements may be tailored to different processes requiring different frequency of measurements.

Another advantage is that the sequence of points in time at which detection of the characteristics is to be made may be adjusted during operation, if required. It may be discovered after a few measurements that the measurements may need to be made more or less frequently, which may then be adjusted for.

Yet another advantage is that a user may trigger activation on-demand. This may be of interest for example if something unexpected is discovered during the monitoring of the process, that requires additional measurement information to be acquired.

According to an embodiment, the wireless communication unit is further configured to:
wirelessly receive a control message for controlling the activation timing for at least one protective membrane of the plurality of protective membranes; and
transfer the control message to the control unit,
thereby allowing wireless control of the activation timing for each protective membranes.

An advantage with this embodiment is that it makes control from a user easier, since there is no need for physical contact to the sensing device. A user may simply input control commands on an external unit which may be done remotely, and the commands may be wirelessly transferred to the sensing device.

According to an embodiment, for each protective membrane, the protective membrane is an electrically breakable membrane, such that the protective membrane breaks upon application of an electric voltage across the protective membrane, and
wherein the control unit is configured to provide the electric voltage across each protective membrane at the respective activation timing.

By way of example, the protective membrane may be made of metal such as Ruthenium. By way of further example, the protective membrane may be made of a patterned metal on top of a thin dielectric film. For example, the protective membrane may preferably comprise Ruthenium, with an underlying layer of titanium or titanium nitride. The Ruthenium may have a thickness in the interval of 10 nm to 200 nm, preferably between 20 nm and 100 nm. The underlying layer of titanium or titanium nitride may typically be thin, as for example having a thickness in the interval of 1-2 nm. By the present arrangement, complete dissolution of the protective membrane may be facilitated.

An advantage of Ruthenium is that it is compatible with CMOS fabrication. Other materials such as gold, silver, platinum, may however, not be compatible with CMOS fabrication or not easily be integrated in a Si CMOS process flow.

According to an embodiment, for each protective membrane, the protective membrane undergoes low-temperature oxidation upon application of an electric voltage across the protective membrane.

By way of example, a voltage may be applied between two or more activation electrodes at the point in time of activation, whereby the protective membrane breaks to expose the sensor to the substance. Upon application of the electric voltage across the protective membrane, the protective membrane may undergo low-temperature oxidation. The electric voltage applied to achieve low-temperature oxidation of the protective membrane may be in the range of 0.5 V to 2 V, such as 0.5 V, 1 V, 1.5 V, or 2 V. Conventional fuze melt on the other hand uses a high electric current through the protective membrane in order to break it. This would cause locally high temperatures that might damage the sensor or the reagent. However, by low-temperature oxidation, breaking the electrically breakable membrane may be done without significant heat development in the vicinity of the sensors or reagent. Low-temperature oxidation of the protective membrane may be fast, as for example it may take a few seconds.

Thus, an advantage with the present embodiment is that an activation process that does not compromise the integrity of the sensors or the reagent may be provided.

According to an embodiment, for each protective membrane, the protective membrane comprises a movable flap, wherein the movable flap is arranged in a closed position and is movable into an open position at which the set of one or more sensors protected by the protective membrane is allowed to be exposed to the substance.

By way of example, the movable flap may be slidable or pivotable.

It serves to mention that the activation may alternatively be passive activation. For example, the protective membrane may be made of a biodegradable material with a well-known degradation rate. Thus, by providing a protective membrane of a biodegradable material with a selected thickness, the degradation time may be selected, thereby providing the activation timing.

According to an embodiment, for each sensor of the plurality of sensors, the sensor is configured to generate an electric signal in response to being exposed to the substance, and wherein the electric signal is dependent on a level of the at least one characteristic of the substance.

An advantage with this embodiment is that electrical signals are easily measured, quantified and digitized for further processing and/or transmission.

According to an embodiment, the sensing device further comprises at least one auxiliary electrode, wherein the sensing device is configured such that the at least one auxiliary electrode is employed in reading out the electric signal from more than one set of one or more sensors, for detection of the at least one characteristic of the substance at more than one point in time.

The at least one auxiliary electrode may be connected to more than one set of sensors. Typically, the at least one auxiliary electrode is connected to several sensors, enabling the at least one auxiliary electrode to be employed in reading out the electric signal from several sensors, at several points in time.

By way of example, the at least one auxiliary electrode may surround the cavities. By way of further example, a surface area of the at least one auxiliary electrode may be at least 3 times as large as a sum of a surface area of all protective membranes of the plurality of protective membranes.

An advantage is that the use of auxiliary electrodes may provide a high level of integration on an integrated circuit. Thus, the sensing device with the plurality of sensors may be provided in a compact format.

Another advantage is that the use of auxiliary electrodes may provide a sensing device with low power consumption.

Further, the compact format as well as the low port consumption may be particularly important for a sensing device for use in a bioreactor or in a body of a human or an animal.

According to an embodiment, for each sensor of the plurality of sensors, the sensor comprises a reagent selected from any one of the groups: enzymes, antibodies, DNA, RNA, aptamers, supramolecular scaffolds, and molecularly imprinted polymers.

According to an embodiment, the at least one characteristic of the substance is one of a protein concentration, a glucose concentration, a lactate concentration, a pH, or a temperature, a cell, a biomarker, a metabolite, or a molecular species.

According to an embodiment, the at least one characteristic of the substance comprises at least a first characteristic and a second characteristic, the first characteristic and the second characteristic being different,
wherein a first subset of the plurality of sensors is configured for detecting the first characteristic, and
wherein a second subset of the plurality of sensors is configured for detecting the second characteristic.

An advantage with this embodiment is that a sensing device configured to detect more than one characteristic may be better suited for monitoring advanced processes. Advanced processes may comprise variation of plural characteristics, thus in order to properly monitoring such a process, detection of more than one characteristic may be required.

According to a second aspect there is provided a capsule for monitoring of at least one characteristic of a substance, the capsule comprising:
a sensing device according to the first aspect;
wherein the capsule is configured to be immersed into the substance, and
wherein the sensing device is arranged in the capsule such that the sensing device is allowed to be exposed to the substance.

The capsule may be placed in or on the substance to be monitored. By way of example, the substance may be a biological material in the form of a liquid. The substance may be placed in a culture flask and the capsule with the sensing device may be placed in the substance of the culture flask to monitor time development of at least one characteristic of the substance.

The capsule may be made to be implanted into the body. Alternatively, the capsule may be made to be swallowed by the human or animal. The capsule may be made of a biocompatible material that is not rejected by a body of a human or animal.

An advantage with this embodiment is that the capsule may protect at least part of the sensing device. Some parts of the sensing device may not be suitable for coming into physical contact with the substance, and may thus need to be protected of the monitoring requires that the sensing device comes into physical contact with the substance.

An advantage of using a capsule made of a biocompatible material that is not rejected by the body is that the capsule and thus the sensing device may be placed in the body of a human or animal without triggering the immune system to cause inflammation, and at the same time keeping the sensing device protected from the environment in the body.

According to a third aspect there is provided a system for monitoring of at least one characteristic of a substance, the system comprising:
a sensing device according to the first aspect;
a communication device external to the sensing device, and configured to receive a data set comprising information from at least one detection of the at least one characteristic of the substance, the communication device being further configured to analyze the data set and/or present results based on the data set to a user.

By way of example, the external communication device may be a computer, a tablet, or a mobile phone. The communication device is configured to receive data sets transmitted from the sensing device of the capsule.

An advantage with this embodiment is that remote monitoring and follow-up of the development status of the process in the substance is enabled.

Another advantage is that it makes control from a user easier. A user may write control commands on an external unit to control the operation of sensing device.

According to a fourth aspect there is provided a method for detection of at least one characteristic of a substance, by a sensing device comprising a plurality of cavities, a plurality of sensors arranged into a plurality of sets of one or more sensors wherein each set of one or more sensors is arranged in a mutually unique cavity of the plurality of cavities, and a plurality of protective membranes wherein each protective membrane of the plurality of protective membranes is arranged to cover an opening of the mutually unique cavity, the method comprising:
preventing, by each protective membrane, the substance from entering the cavity, thereby protecting a mutually unique set of one or more sensors of the plurality of sets from being exposed to the substance;
providing a different activation timing for different protective membranes among the plurality of protective membranes, to allow the set of one or more sensors protected by the different protective membranes to be exposed to the substance, whereby different sets of one or more sensors are exposed to the substance at different points in time; and
detecting, by the sets of one or more sensors, the at least one characteristic of the substance at multiple time points.

According to an embodiment, the method further comprises, for each sensor of the plurality of sensors:
generating an electric signal in response to being exposed to the substance, wherein the electric signal is dependent on a level of the at least one characteristic of the substance;
reading out the electric signal; and
determining, based on the electric signal, a value of the at least one characteristic of the substance.

Effects and features of the second, third and fourth aspects are largely analogous to those described above in connection with the first aspect. Embodiments mentioned in relation to the first aspect are largely compatible with the second, third and fourth aspects. It is further noted that the inventive concepts relate to all possible combinations of features unless explicitly stated otherwise.

Other objectives, features and advantages of the present inventive concept will appear from the following detailed disclosure, from the attached claims as well as from the drawings.

### Brief descriptions of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1A schematically illustrates a sensing device for detection of at least one characteristic of a substance.
Fig. 1B schematically illustrates the arrangement of a cavity with a sensor of the sensing device in more detail.
Fig. 1C schematically illustrates an alternative arrangement of a cavity with a set of two sensors.
Fig. 1D schematically illustrates another alternative arrangement of cavities each with a single sensor.
Fig. 2 schematically illustrates a sensing device for detection of at least one characteristic of a substance, the sensing device further comprises two auxiliary electrodes.
Fig. 3 schematically illustrates a capsule comprising a sensing device as part of a system for monitoring of at least one characteristic of a substance.
Fig. 4 illustrates a schematic block diagram shortly summarizing the method for detection of at least one characteristic of a substance.

### Detailed description

In cooperation with attached drawings, the technical contents and detailed description of the present inventive concept are described thereinafter according to a preferable embodiment, being not used to limit the claimed scope. This inventive concept may be embodied in many different forms and should not be construed as limited to the embodiments set forth herein; rather, these embodiments are provided for thoroughness and completeness, and fully convey the scope of the inventive concept to the skilled person.

In the present description the substance being monitored by the sensing device is exemplified as being a biological material. However, it should be understood that the substance is not limited to being a biological material, but may equally well be another type of substance, as for example air or other gases, water or other liquids, soil, synthetic substances, or chemicals.

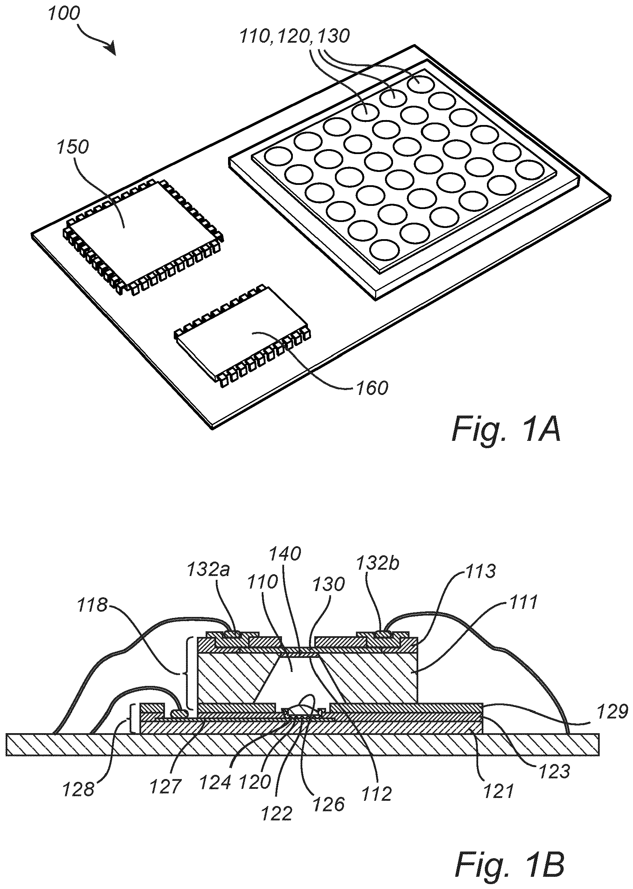

Fig. 1A schematically illustrates a sensing device 100 for detection of at least one characteristic of a substance. The sensing device 100 is in the form of an integrated circuit, however, the sensing device 100 is not limited to being in the form of an integrated circuit.

The sensing device 100 is configured for coming into physical contact with the substance, in order to detect the characteristic of the substance. Given as non-limiting examples, in case the substance is in the form of a liquid, the sensing device 100 may come into physical contact with the substance by being submerged into or floating in the substance. Given as another non-limiting example, the sensing device 100 may be a device configured to be implanted into a body of a human or an animal.

The sensing device 100 comprises a plurality of cavities 110. In the present example, the plurality of cavities 110 are arranged in rows and columns as an array of cavities 110. The sensing device 100 further comprises a plurality of sensors 120 configured for detecting the characteristic of the substance. The plurality of sensors 120 are arranged into a plurality of sets of one or more sensors 120. Each set of sensors 120 is arranged in a mutually unique cavity 110, the opening of which is covered by a protective membrane 130.

Fig. 1B schematically illustrates the arrangement of a cavity 110 with a set 122 of sensors 120 in more detail. As illustrated in Fig. 1B, the set 122 of sensors 120 may comprise a single sensor 120. However, it should be understood that the set 122 of sensors 120 may alternatively comprise more than one sensor 120.

By way of example, the characteristic of the substance which the sensor 120 is configured to detect, may be a protein concentration, a glucose concentration, a lactate concentration, a pH, a temperature, a dissolved gas, a cell, a biomarker, a metabolite, or a molecular species.

The sensor 120 may be configured to generate an electric signal, such as an electric current or voltage, in response to being exposed to the substance. The electric signal may be dependent on a level of the characteristic of the substance. The electric signal may be read via electrodes 124.

As illustrated in Fig. 1B, the sensor 120 comprises a reagent 126. A reagent may be required for biosensing reactions to occur and to generate an electric signal. A sensor 120 comprising a reagent may be a one-time use sensor, since the reagent may be consumed upon exposure to the substance. The type of reagent depends on what characteristic of the substance that is to be detected by the sensor 120. Given as non-limiting examples, the reagent may be selected from any one of the groups: enzymes, antibodies, DNA, RNA, aptamers, supramolecular scaffolds, and molecularly imprinted polymers. The selected reagent should be capable of binding a targeted characteristic.

A protective membrane 130 is arranged to cover the opening of the cavity 110. The protective membrane 130 prevents the substance from entering the cavity 110. Thereby, the protective membrane 130 may protect the sensor 120 from being exposed to the substance. Thus, as long as the protective membrane 130 is intact, the reagent may remain in the enclosed cavity, thereby ensuring the reagents integrity and stability, even if the sensing device 100 has engaged in physical contact with the substance.

The sensing device 100 is configured for providing an activation timing for the protective membrane 130, to allow the sensor 120 protected by the protective membrane 130 to be exposed to the substance. At the point in time at which the sensor 120 is intended to make a measurement to detect the characteristic of the substance, the protective membrane 130 is to be opened to allow the sensor 120 protected by the protective membrane 130 to be exposed to the substance. In the sensing device 100 illustrated here, the protective membrane 130 is an electrically breakable membrane. Thus, the protective membrane 130 will break upon application of an electric voltage across the protective membrane 130. In order to provide an electric voltage, the protective membrane 130 is connected to activation electrodes 132a, 132b. By the present arrangement, a voltage may be applied between the activation electrodes 132a, 132b at the point in time for activation, whereby the protective membrane 130 breaks to expose the sensor 120 to the substance. More specifically, upon application of the electric voltage across the protective membrane 130, the protective membrane 130 undergoes low-temperature oxidation. Although Fig. 1B illustrates two activation electrodes 132a, 132b, it should be understood that the protective membrane 130 may alternatively be connected to only a single activation electrode132a or 132b. A single activation electrode may be sufficient for low-temperature oxidation of the protective membrane 130. By way of example, the electric voltage applied to achieve low-temperature oxidation of the protective membrane 130 may be 0.5 V, 1 V, 1.5 V, or 2 V. By way of further example, low-temperature oxidation of the protective membrane 130 may take about 10 s, 20 s, 30 s, 40 s, 50 s, or 60 s. The low temperature oxidation does not involve any significant heat development to other parts such as to the sensor 120 or the reagent 126. In the manner described above, an activation of the sensor 120 may be provided without damaging the sensor and/or the reagent. Damage to the sensor 120 and/or the reagent 126 as a consequence of heat may otherwise compromise the quality and accuracy of the measurement. Therefore, preventing such damage may ensure maintained measurement quality and accuracy.

However, by two activation electrodes 132a, 132b, thermal disruption of the protective membrane 130 may be additionally provided, allowing a greater current through the membrane to heat and potentially melt the protective membrane 130.

It serves to mention that also alternative manners in which to activate the sensor may be conceivable. For example, the protective membrane 130 may comprise a movable flap initially arranged in a closed position. Upon activation, the movable flap may be moved into an open position at which the sensor protected by the protective membrane 130 is allowed to be exposed to the substance. By way of example, the movable flap may be slidable or pivotable.

Further, activation may alternatively be a passive activation. For example, the protective membrane 130 may be made of a biodegradable material. The biodegradable material may have a well-known degradation rate from the point in time at which the protective membrane comes into contact with the substance may. Thus, by providing a protective membrane 130 of a biodegradable material with a selected thickness, the degradation time may be selected, thereby providing the activation timing.

The sensing device may optionally comprise a plurality of semi-permeable membranes 140. Each semi-permeable membrane 140 may be arranged to cover the opening 112 of the mutually unique cavity 110. As illustrated in Fig. 1B, the semi-permeable membrane 140 may be arranged on the inside of the cavity 110 with respect to the protective membrane 130, however, the semi-permeable membrane 140 may alternatively be arranged on the outside of the cavity 110 with respect to the protective membrane 130.

The semi-permeable membrane 140 is configured to allow passage of a liquid through the semi-permeable membrane 140 and to prevent passage a solid substance through the semi-permeable membrane 140. At the activation timing of the protective membrane 130, the protective membrane 130 breaks and allows the sensor 120 to be exposed to the substance so as to provide detection of the characteristic of the substance. The substance may be in the form of a liquid comprising also a solid substance. For example, in case the substance is a biological material, the solid substance may be in the form of cells in the liquid. In at least some applications a measurement of a characteristic of the substance using the sensor 120 may be performed on the liquid coming into physical contact with the sensor 120, whereas the solid substance, if coming into physical contact with the sensor 120, may introduce interference or errors in the measurement. By providing the semi-permeable membrane 140 to cover the opening 112 of the cavity 110 protection of the sensor 120 from being exposed to the solid substance may be provided, while still allowing the sensor 120 to be exposed to the liquid. In the manner described above, a sensing device 100 configured to detect a characteristic of the substance by being exposed to the liquid of the substance yet reducing or eliminating interference or errors otherwise introduced by solid substances may be provided.

It serves to mention that when manufacturing the sensor device 100, the sensor portion 128 and cavity/membrane portion 118 may be manufactured separately. Applying the protective membrane 130 to cover the opening 112 of the cavity 110 may involve harsh processing steps than may risk damaging the sensor 120, or if a reagent 126 is used, it may risk damaging the reagent 126. However, by manufacturing the cavity/membrane portion 118 separately from the sensor portion 128, the protective membrane 130 may be applied to cover the opening 112 of the cavity 110 without the sensor 120 being present during the process. In this manner, the process of applying the protective membrane 130 does not risk damaging the sensor 120. After separate manufacturing of the two portions 118, 128, the two portions 118, 128 may be combined and assembles into a complete sensing device 100.

As an option, an adhesive layer (129) may be applied on sensor portion 128 or on the cavity/membrane portion 118.

The cavity/membrane portion 118 may be provided on a substrate 111. By way of example, the substrate 111 may be made of Si. By way of further example, the substrate 111 may have a thickness of about 500 µm, which provides the depth of the cavity 110. Given only as an example, the cavity 110 may have a depth of preferably 400 µm. The cavity 110 may have a diameter of about 200 µm at the side facing the sensor portion 128. Further, the cavity 110 may have a smaller diameter of about 50 µm at the side of the opening 112. However, although Fig. 1B illustrates a cavity having side walls being inclined or slanted with respect to the sensor surface, it is equally conceivable that the cavity 110 comprises side walls being perpendicular to the sensor surface.

The substrate 111 may have a top layer 113 which is typically significantly thinner than the thickness of the substrate 111. By way of example, the top layer 113 may be made of SiOₓ.

The protective membrane 130 may be provided to cover the opening 112 of the cavity 110, in between the substrate 111 and the top layer 113. By way of example, the protective membrane 130 may be made of metal. Given as non-limiting examples, the protective membrane 130 may be made of Ruthenium or of a layer structure of a 30 nm thick layer of Ruthenium, a 300 nm thick layer of Titanium, and another 30 nm thick layer of Ruthenium. The protective layer 130 may be connected to activation electrodes 130a, 130b made of Aluminium.

The sensor portion 128 may be provided on a substrate 121. By way of example, the substrate 121 may be made of Si.

The substrate 121 may be provided with a top layer 123. By way of example, the top layer 123 may be made of SiOₓ. By way of further example, the top layer 123 may be made of a layer structure comprising layers of SiOₓ, and/or Si. The adhesive layer 129 may be applied either on sensor portion 128 or on the cavity/membrane portion 118, to bond the two portions together under mild condition for the bioreceptor, e.g. room temperature, without requiring high temperature and/or pressure.

The substrate 121 may be provided with active circuitry, e.g., for processing signals from the sensor 120.

By way of example, the sensor 120 may be made of Ruthenium and/or Aluminium. By way of further example, the electrode 124 may be made of Ruthenium and/or Aluminium. Further, a layer of aluminium forming an aluminium track 127 may be provided, onto which the sensor 120 may be deposited.

In Fig. 1B, the connections to the protective membrane 130 and to the sensor 120 are illustrated as simple wire bonds. However, it should be understood that the connections may be provided in a number of other manners. By way of example, the connections to the protective membrane 130 and to the sensor 120 may be provided as direct connections as a metal through substrates 111 and 121. Such direct connections may be connected to one or more integrated switches on the substrates.

Returning now to Fig. 1A, the sensing device 100 may further comprise a control unit 150. The control unit 150 is configured to control the activation timing for each protective membrane 130. In the present example, the control unit 150 is configured to control the activation timing by providing an electric voltage across each protective membrane 130 at the respective activation timings. By the present arrangement, the control unit 150 may provide a different activation timing for different protective membranes 130. Consequently, different sensors 120 may be exposed to the substance at different points in time, thereby providing detection of the characteristic of the substance at multiple time points.

By the present arrangement, the respective sensors 120 and optionally also the respective reagents are not used until the activation and may remain stable and protected until the intended time of use. Thus, the arrangement may provide a sensing device 100 that may be stable and long-time reusable and that may be used for multiple time point sensing events. Put differently, a sensing device 100 comprising an array of cavities 110 and sensors 120 with respective protective membranes 130 for actuation may be used to create time lapse monitoring such that, for each activation time point sensor 120 of a subset of sensors may be activated.

By way of example, the control unit 150 may be configured for allowing programable control of the activation timing for each protective membrane 130. Thus, a user may be able to program when and how the respective cavities 110 are to be activated to make a measurement. The programming may comprise providing a specific time delay between activation of two consecutive cavities. The time delay may be the same for all consecutive cavities 110, or the time delay may alternatively be individually set for each pair of consecutive cavities 110.

It is conceivable that the sensing device 100 may alternatively reprogram the activation timings based on previously measured data. For example, activation may be provided with a certain frequency when the measured value is on side of a predefined threshold. Once a measurement is made detecting that the value has now passed to the other side of the threshold, the activation timing may be altered for coming activations such that activation occurs more or less frequently.

The sensing device 100 may further comprise a wireless communication unit 160. The wireless communication unit may be configured to wirelessly transmit a data set comprising information from at least one detection of the characteristic of the substance. The data set may be transmitted from the sensing device 100 to an external device (not illustrated here). The external device may be for example a computer, a tablet, or a mobile phone. The external device may be able to analyze and/or present the data to a user, thereby facilitating monitoring of the status of the substance.

The wireless communication unit 160 may be further configured to allow wireless programming of the activation timings. The wireless communication unit 160 may thus be configured to wirelessly receive a control message for controlling the activation timing for at least one protective membrane 130. The wireless communication unit 160 may be configured to transfer the control message to the control unit 150. The control unit 150 may further be configured to adjust the activation timing in accordance with the received control message, thereby allowing wireless control of the activation timing for each protective membranes 130.

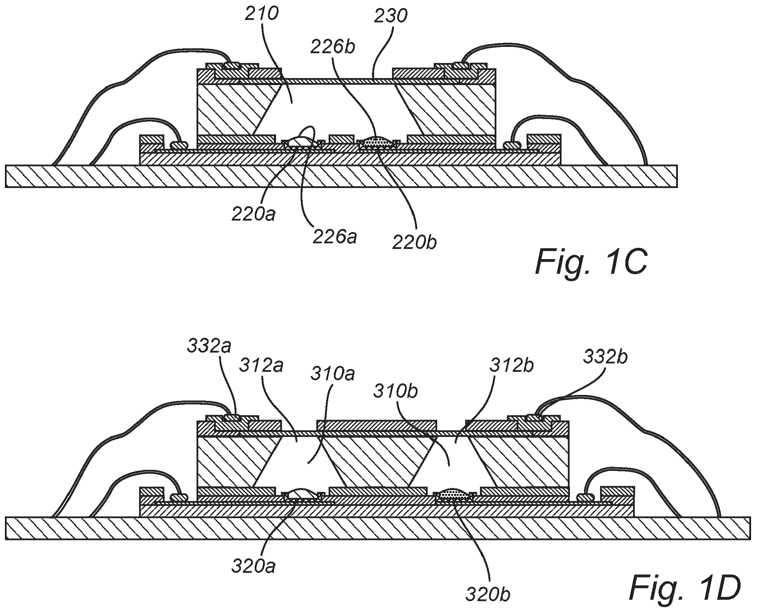

Fig. 1C schematically illustrates an alternative arrangement of a cavity 210 with a set 222 of sensors 220a, 220b. The alternative arrangement of the cavity 210 and the set 222 of sensors 220 shares some of the features with cavity 110 and set 122 described in relation to Fig. 1B, the details of which are not repeated here.

As illustrated in Fig. 1C, the set 222 of sensors 220a, 220b may comprise two sensors 220a, 220b, i.e. a first sensor 220a and a second sensor 220b. the first sensor 220a and the second sensor 220b may optionally comprise a reagent 226a, 226b, respectively.

In the present arrangement, the first sensor 220a is configured for detecting the characteristic of the substance, similar to the sensor 120 of Fig. 1B. The second sensor 220b may be configured for detecting a background signal. The background signal detected by the second sensor 220b may be subtracted from the signal detected by the first sensor 220a, in order to improve the accuracy of the detection of the characteristic of the substance.

The opening 212 of the cavity is covered by a protective membrane 230, thus at the activation timing when the protective membrane 230 breaks the first sensor 220a and the second sensor 220b are activated simultaneously.

Fig. 1D schematically illustrates another alternative arrangement of cavities 31 0a, 310b each with a single sensor 320a, 320b. This alternative arrangement shares some of the features with cavity 110 and set 122 described in relation to Fig. 1B, and with cavity 210 and set 222 described in relation to Fig. 1C, the details of which are not repeated here. The two cavities 31 0a, 310b are separate cavities, however their respective openings 312a, 312b are covered by a joint protective membrane 330.

By applying a voltage between the activation electrodes 332a, 332b at the point in time for activation, the protective membrane 330 will breaks to expose the two sensors 320a, 320b to the substance substantially simultaneously. In this manner, the sensing device may be configured for providing the same activation timing for sensors 320a, 320b in two cavities 310a, 310b, to allow the sensors 320a, 320b protected by the protective membrane 330 to be exposed to the substance at a same point in time.

By way of example, the present arrangement may provide detection of the characteristic of the substance simultaneously by the two sensors 320a, 320b. Simultaneous detection of the characteristic by two sensors 320a, 320b may provide a more accurate result.

Alternatively, the sensor 320a may form part of a first subset of the plurality of sensors, and the sensor 320b may form part of a second subset of the plurality of sensors. The first subset of the plurality of sensors may be configured for detecting a first characteristic of the substance, and the second subset of the plurality of sensors may be configured for detecting the second characteristic. Thus, in the manner described above, a sensing device configured for simultaneous detection of the first characteristic and the second characteristic may be provided.

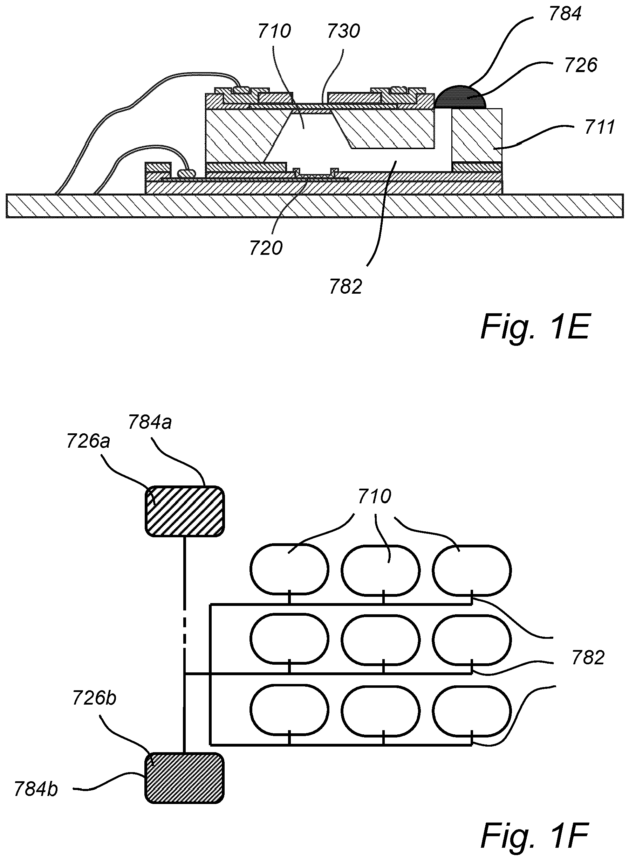

Fig. 1E schematically illustrates an alternative arrangement for providing the reagent to the sensor 720 in the cavity 710. The present example is based on the cavity 110 as illustrated in relation to Fig. 1B, however it should be understood that the arrangement of providing the reagent to the sensor may be equally applicable to any type of cavity, such as cavity 210 or cavity 310.

Activation is provided to rupture the protective membrane 730, whereby the sensor 720 is exposed to the substance. As illustrated in Fig. 1E, the cavity 710 may be connected to a microfluidic channel 782. By way of example, the microfluidic channel 782 may be provided in the substrate 711. Reagents 726 may be stored in a reservoir 784. The reagents 726 may be delivered to the cavity 710 and onto the sensor 720 surface to enable sensitive sensor readout, by being guided through the microfluidic channel 782. By way of example, delivery of reagents 726 may be provided by valves and pumps (not illustrated here) that might be integrated with the respective cavity 710. Given only as an example, the arrangement may comprise a control unit further configured to control the delivery of the reagents 726, e.g. by controlling the pumps and valves.

The reservoir 784 may be affixed to the chip surface and/or in the system. By way of example, the reagent 726 may be dispensed into the microfluidic channel 782 by subjecting the reservoir 784 to a pressure change sufficient to displace the contained liquid into the microfluidic channel 782. The reagent 726 may flush the substance out of the cavity 710 as well as the sensor 720 surface. By the present arrangement, non-specifically bound analytes and interferents may be removed to results in a more specific, sensitive and accurate measurements of the specifically bound analytes.

Achieving sensitive detection of analytes via surface bound, heterogeneous, assay based on affinity reactions (e.g. immunoassay) may require washing of the excess of unbound analytes and potentially interfering species to reduce the contribution of non-specific binding to the overall signal (i.e. bioassay noise). This may be particularly important when trying to detect trace amounts of analytes, such as cell secreted biomarkers in culture media, or toxins and contaminants in the environment. It is therefore advantageous to be capable of delivering additional reagents, such as wash buffer, or detection reagents (e.g. fluorescent or enzymatic labels, substrates) to the sensor 720.

It serves to mention that the sensor 720 may be arranged directly under the protective membrane 730, as illustrated in Fig. 1E, or alternatively the sensor 720 may be arranged within the microfluidic channel 782 linking the cavity 720 to the reagent reservoir 784.

Fig. 1F schematically illustrates an arrangement for providing reagents 726a, 726b to the sensors in a plurality of cavities 710. The reagents 726a, 726b may be delivered to the cavities 710 and onto the sensor surfaces by being guided through the microfluidic channels 782 from the respective reservoirs 784a, 784b. A plurality of reagents may be used to achieve the required sensor performance, thus a plurality of reservoirs 784a, 784b may be provided.

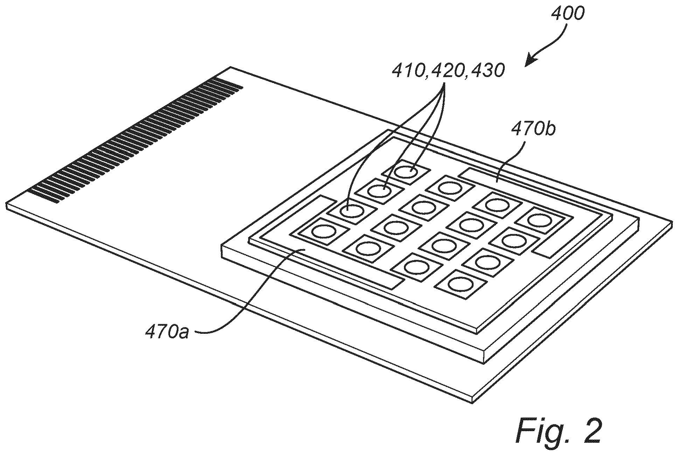

Fig. 2 schematically illustrates a sensing device 400 for detection of at least one characteristic of a substance. The sensing device 400 shares some of the features with sensing device 100 described in relation to Fig. 1A, the details of which are not repeated here.

The sensing device 400 comprises a plurality of cavities 410 and a plurality of sensors 420 arranged into a plurality of sets of one or more sensors 420. Each set of sensors 420 is arranged in a mutually unique cavity 410, the opening of which is covered by a protective membrane 430.

The sensing device 400 is in the form of an integrated circuit. The sensing device 400 further comprises two auxiliary electrodes 470a, 470b. The two auxiliary electrodes 470a, 470b are respectively connected to more than one set of sensors 420. Typically, each auxiliary electrode 470a, 470b is connected to several sensors, such that the auxiliary electrodes 470a, 470b may be employed in reading out the electric signal from several sensors 420, at several points in time.

Auxiliary electrodes 470a, 470b may provide a high level of integration on the integrated circuit. By the present arrangement, a sensing device 400 with a plurality of sensors may be provided in a compact format. By way of example, the auxiliary electrodes may have a width of about 100 µm. By way of further example, the electrodes may be made of Ruthenium.

Further, the use of auxiliary electrodes 470a, 470b may provide a sensing device 400 having low power consumption. The compact format as well as the low port consumption may be particularly important for a sensing device 400 for use in a bioreactor or in a body of a human or an animal.

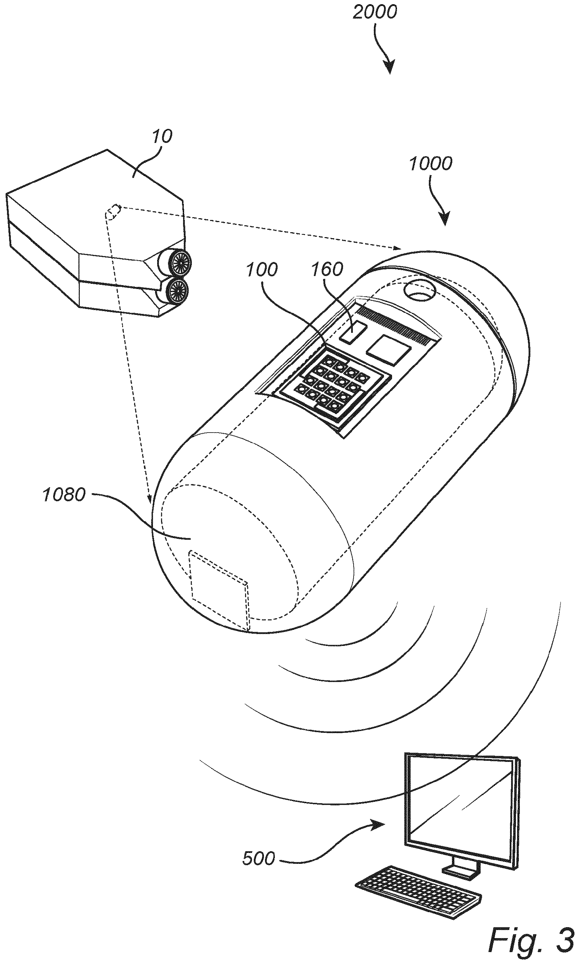

Fig. 3 schematically illustrates a capsule 1000 comprising a sensing device 100 as part of a system 2000 for monitoring of at least one characteristic of a substance. Although the capsule 1000 is here illustrated and described as comprising the sensing device 100, it should be understood that as an alternative to the sensing device 100, the capsule 1000 may equally well comprise the sensing device 400.

The capsule 1000 may further comprise a power source 1080, such as a battery, to power the circuits of the sensing device 100. However, it is conceivable that the poser source 1080 alternatively forms part of the sensing device 100.

The capsule 1000 is a capsule for monitoring of at least one characteristic of a substance, into which the capsule 1000 is immersed. In the present example, the capsule may be used for detection of characteristics of a substance being a biological material. However, it should be understood that the capsule 1000 may alternatively be used in other application areas and thus may be configured for detecting other characteristics.

By way of example, the capsule 1000 may be used for monitoring production batches involving biological processes, such as production of vaccines, antibody medicine and cell therapies. Characteristics of interest in such processes of biological material may be characteristics related to metabolic activity, such as glucose or lactate concentrations, or it could be characteristics such as concentrations of certain proteins in the batch. Monitoring of such parameters may provide information on product quality of the biological medicine, information about potential contaminations, and information on growth. The capsule 1000 may be configured for monitoring one or more of said characteristics of the biological material into which it is immersed.

The biological material may be arranged in a culture flask 10, and the capsule 1000 may be placed therein in order to perform the monitoring. The sensing device 100 is arranged in the capsule 1000 such that the sensing device is allowed to be exposed to the substance. By way of example, the sensing device 100 may be arranged at a surface of the capsule such that biological material surrounding the capsule may come into contact with the sensing device 100. By the present arrangement, the capsule may provide in situ autonomous measurements of the characteristic by activation of different sensors at different points in time.

The capsule 1000 forms part of a system 2000 for monitoring of at least one characteristic of a substance. In addition to the capsule 1000, the system 2000 further comprises a communication device 500 external to the capsule 1000 and thus external to the sensing device 100. By way of example, the external communication device 500 may be a computer, a tablet, or a mobile phone. The communication device 500 is configured to receive data sets transmitted from the sensing device 100 of the capsule 1000. The data sets may comprise information from at least one detection of the characteristic of the substance. Since the sensing device 100 comprises a wireless communication unit 160, the data sets may be wirelessly transmitted from the sensing device 100 to the external communication device 500, without the need for extracting neither a sample of the substance nor the capsule 1000 from the culture flask. The communication device 500 may be configured to analyze the data sets and/or present results based on the data set to a user. Thus, by the present arrangement, in situ autonomous measurements of the characteristic of the substance in the culture flask may be provided by activation of different sensors at different points in time, to create time lapse monitoring without the need for extracting samples from the culture flask.

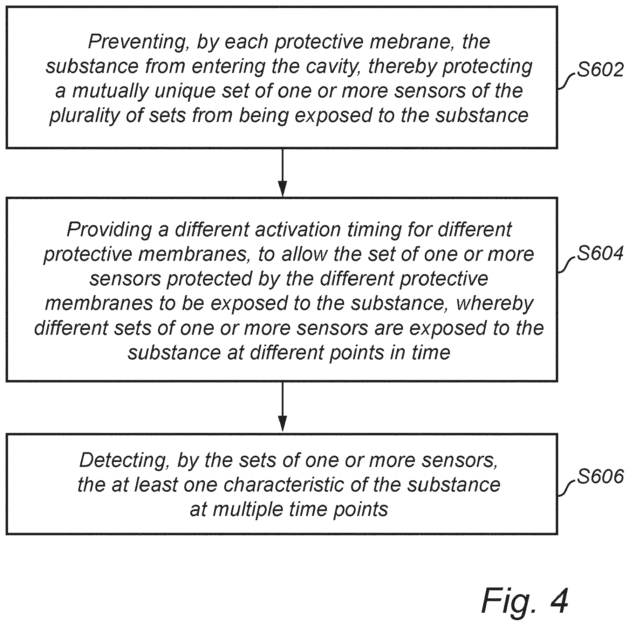

Fig. 4 illustrates a schematic block diagram shortly summarizing the method for detection of at least one characteristic of a substance, by a sensing device comprising a plurality of cavities, a plurality of sensors arranged into a plurality of sets of one or more sensors wherein each set of one or more sensors is arranged in a mutually unique cavity of the plurality of cavities, and a plurality of protective membranes wherein each protective membrane of the plurality of protective membranes is arranged to cover an opening of the mutually unique cavity. It should be understood that the steps of the method, although listed in a specific order herein, may be performed in any order suitable.

The method may comprise preventing S602, by each protective membrane, the substance from entering the cavity, thereby protecting a mutually unique set of one or more sensors of the plurality of sets from being exposed to the substance.

The method may comprise providing S604 a different activation timing for different protective membranes among the plurality of protective membranes, to allow the set of one or more sensors protected by the different protective membranes to be exposed to the substance, whereby different sets of one or more sensors are exposed to the substance at different points in time.

The method may comprise detecting S606, by the sets of one or more sensors, the at least one characteristic of the substance at multiple time points.

The method may further comprise protecting the plurality of sensors during a sterilization process and/or during storage of the sensing device.

The method may further comprise generating, for each sensor of the plurality of sensors, an electric signal in response to being exposed to the substance, wherein the electric signal is dependent on a level of the at least one characteristic of the substance.

The method may further comprise, for each sensor of the plurality of sensors, reading out the electric signal.

The method may further comprise, for each sensor of the plurality of sensors, determining, based on the electric signal, a value of the at least one characteristic of the substance.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A sensing device for detection of at least one characteristic of a substance, the sensing device comprising:
a plurality of cavities, wherein each cavity of the plurality of cavities comprises an opening;
a plurality of sensors configured for detecting the at least one characteristic of the substance, wherein the plurality of sensors is arranged into a plurality of sets of one or more sensors, and wherein each set of one or more sensors is arranged in a mutually unique cavity of the plurality of cavities;
a plurality of protective membranes, wherein each protective membrane of the plurality of protective membranes is arranged to cover the opening of the mutually unique cavity, so as to prevent the substance from entering the cavity, thereby protecting a mutually unique set of one or more sensors of the plurality of sets from being exposed to the substance;
wherein the sensing device is configured for providing a different activation timing for different protective membranes among the plurality of protective membranes, to allow the set of one or more sensors protected by the different protective membranes to be exposed to the substance, whereby different sets of one or more sensors are exposed to the substance at different points in time, for providing detection of the at least one characteristic of the substance at multiple time points.

2. The sensing device according to claim 1, further comprising a plurality of semi-permeable membranes, wherein each semi-permeable membrane of the plurality of semi-permeable membranes is arranged to cover the opening of the mutually unique cavity, wherein each semi-permeable membrane is configured to allow passage of a liquid through the semi-permeable membrane and to prevent passage a solid substance through the semi-permeable membrane, thereby protecting the mutually unique set of one or more sensors of the plurality of sets from being exposed to the solid substance.

3. The sensing device according to any one of claims 1 or 2, wherein each set of one or more sensors comprises a first sensor configured for detecting the at least one characteristic of the substance, and a second sensor configured for detecting a background signal.

4. The sensing device according to any one of the preceding claims, further comprising a wireless communication unit configured to:
wirelessly transmit, from the sensing device to an external device, a data set comprising information from at least one detection of the at least one characteristic of the substance.

5. The sensing device according to any one of the preceding claims, further comprising a control unit configured to control the activation timing for each protective membrane, thereby controlling the points in time at which the set of one or more sensors protected by the different protective membranes is allowed to be exposed to the substance.

6. The sensing device according to claim 5, wherein the control unit is further configured for allowing programmable control of the activation timing for each protective membrane.

7. The sensing device according to any one of claims 5 or 6, when dependent on claim 4, wherein the wireless communication unit is further configured to:
wirelessly receive a control message for controlling the activation timing for at least one protective membrane of the plurality of protective membranes; and
transfer the control message to the control unit,
thereby allowing wireless control of the activation timing for each protective membranes.

8. The sensing device according to any one of claims 5 to 7, wherein, for each protective membrane, the protective membrane is an electrically breakable membrane, such that the protective membrane breaks upon application of an electric voltage across the protective membrane, and
wherein the control unit is configured to provide the electric voltage across each protective membrane at the respective activation timing.

9. The sensing device according to claim 8, wherein, for each protective membrane, the protective membrane undergoes low-temperature oxidation upon application of an electric voltage across the protective membrane.

10. The sensing device according to any one of the preceding claims, wherein, for each sensor of the plurality of sensors, the sensor is configured to generate an electric signal in response to being exposed to the substance, and wherein the electric signal is dependent on a level of the at least one characteristic of the substance.

11. The sensing device according to claim 10, further comprising at least one auxiliary electrode, wherein the sensing device is configured such that the at least one auxiliary electrode is employed in reading out the electric signal from more than one set of one or more sensors, for detection of the at least one characteristic of the substance at more than one point in time.

12. The sensing device according to any one of the preceding claims, wherein, for each sensor of the plurality of sensors, the sensor comprises a reagent selected from any one of the groups: enzymes, antibodies, DNA, RNA, aptamers, supramolecular scaffolds, and molecularly imprinted polymers.

13. A capsule for monitoring of at least one characteristic of a substance, the capsule comprising:
a sensing device according to any of the preceding claims;
wherein the capsule is configured to be immersed into the substance, and
wherein the sensing device is arranged in the capsule such that the sensing device is allowed to be exposed to the substance.

14. A system for monitoring of at least one characteristic of a substance, the system comprising:
a sensing device according to any one of claims 1 to 12;
a communication device external to the sensing device, and configured to receive a data set comprising information from at least one detection of the at least one characteristic of the substance, the communication device being further configured to analyze the data set and/or present results based on the data set to a user.

15. A method for detection of at least one characteristic of a substance, by a sensing device comprising a plurality of cavities, a plurality of sensors arranged into a plurality of sets of one or more sensors wherein each set of one or more sensors is arranged in a mutually unique cavity of the plurality of cavities, and a plurality of protective membranes wherein each protective membrane of the plurality of protective membranes is arranged to cover an opening of the mutually unique cavity, the method comprising:
preventing, by each protective membrane, the substance from entering the cavity, thereby protecting a mutually unique set of one or more sensors of the plurality of sets from being exposed to the substance;
providing a different activation timing for different protective membranes among the plurality of protective membranes, to allow the set of one or more sensors protected by the different protective membranes to be exposed to the substance, whereby different sets of one or more sensors are exposed to the substance at different points in time; and
detecting, by the sets of one or more sensors, the at least one characteristic of the substance at multiple time points.
